# EUROPEAN PATENT SPECIFICATION

(11) **EP 4 413 188 B1**
(45) Date of publication and mention of the grant of the patent: **28.10.2026**
(21) Application number: 22809236.7
(22) Date of filing: 03.10.2022
(51) Int. Cl.: D03D 1/00, D03D 11/02, A61F 2/06

(54) **INTEGRALLY WOVEN MANIFOLDS AND METHODS FOR FORMING**
INTEGRAL GEWEBTE VERTEILER UND VERFAHREN ZUR HERSTELLUNG
COLLECTEURS TISSÉS D'UN SEUL TENANT ET PROCÉDÉS DE FORMATION

(30) Priority: 04.10.2021 US 202163251941 P
(43) Date of publication of application: 14.08.2024
(73) Proprietor: The Secant Group, LLC, Telford, PA 18969 (US)
(72) Inventor: WEBER, Amanda K., Macungie, PA 18062 (US); AMIN, Swati, Schwenksville, PA 19473 (US); METZGER, Andrew, Lafayette Hill, PA 19444 (US); GABRIELE, Peter, Frisco, TX 75034 (US)
(74) Representative: Prinz & Partner mbB
(86) International application number: PCT/US2022/045551
(87) International publication number: WO 2023/059556

(56) References cited:
- DE-T2- 69 728 268
- DE-T2- 69 828 977
- US-A1- 2020 205 961
- US-B2- 8 540 764

## Description

### FIELD OF THE INVENTION

This application is directed to woven manifolds and methods for forming woven manifolds. More particularly, this application is directed to integrally woven manifolds and methods for forming integrally woven manifolds.

### BACKGROUND OF THE INVENTION

Renal artery stenosis is the narrowing of arteries that transport blood to kidneys. In order to prevent inadequate blood flow or blockages to the kidneys, a renal artery bypass surgery may be required. Current state of the art prosthetic implants which are used require suturing multiple grafts together in order to accommodate the near 90° angle between the aorta and the renal arteries. In order to build the current grafts, a section of the aortic graft is typically punctured, cut, or burst so as to create a hole to which extension grafts are fixated.

US 2020 / 0 205 961 A1 describes a textile engineered prosthetic comprising a continuous tube having a body portion and a bifurcated portion extending from the body portion. At least one band of increased thickness is formed over a portion of the continuous tube, which forms a biomimetic surface.

DE 697 28 268 T2 shows a flat-woven implantable tubular prosthesis having warp yarns and fill yarns comprising first and second spaced apart portion which define therebetween a transition tubular wall extent. Said tubular prosthesis further includes a weaving pattern along said transition tubular wall extent, said weaving pattern having a gradual change in the number of warp yarns to provide a seamless transition between said first portion and said second portion.

US 8 540 764 B2 discloses an endovascular prosthesis comprising a tubular body including a graft material. The prosthesis further comprises a mobile external coupling at an opening in the graft material, the mobile external coupling extending outwardly from the tubular body. Said mobile external coupling is used to align the endovascular prothesis such that the mobile external coupling is positioned at a branch vessel. Then, the mobile external coupling is used to deliver a branch stent-graft into the branch vessel.

There is a need for woven manifolds, in particular for prosthetic implants for renal artery stenosis, which do not require suturing multiple grafts together and which do not have the structural infirmities brought about by puncturing, cutting, or bursting a graft.

### BRIEF DESCRIPTION OF THE INVENTION

The object of the invention is solved by an integrally woven manifold according to claim 1 and a method for forming an integrally woven manifold according to claim 13.

### BRIEF DESCRIPTION OF THE DRAWINGS

FIG. 1 illustrates an integrally woven manifold in the form of an endovascular aneurysm repair graft having a primary lumen and a secondary lumen, according to an embodiment of the present disclosure.
FIG. 2 illustrates an integrally woven manifold in the form of an endovascular aneurysm repair graft having a primary lumen, a secondary lumen, and a tertiary lumen, according to an embodiment of the present disclosure.
FIG. 3 illustrates an integrally woven manifold in the form of an aortic arch graft having a primary lumen, a secondary lumen, a tertiary lumen, and a quaternary lumen, prior to the removal of floating ends, according to an embodiment of the present disclosure.
FIG. 4 illustrates a sectional view of the integrally woven manifold of FIG. 3 following removal of the floating ends, according to an embodiment of the present disclosure.

Wherever possible, the same reference numbers will be used throughout the drawings to represent the same parts.

### DETAILED DESCRIPTION OF THE INVENTION

Embodiments of the present disclosure, in comparison to woven manifolds and methods for forming woven manifolds lacking one or more of the features of the present invention, may decrease costs of production, decrease potential points of failure in the resulting devices, provide practical customizability, extend service life, or combinations thereof. Textile patterning may be used to create arm extensions rather than attaching two or more separate woven constructs together with stitching. In one embodiment, an integrally woven multi-lumen graft is formed by textile patterning rather than suturing two or more woven graft structures together to create a multi-branched graft. Unlike existing woven manifolds which require building multipiece structures, present embodiments include integrally woven manifolds such as single unit graft components with radial extensions. By simplifying and eliminating the cutting and fixation processes of current multipiece structure options, lower cost, quicker production, and lower risk associated with handling per component may be realized. Further, interlacing and looping from a primary lumen (such as an aortic region) to secondary lumens (such as arm extension regions) may provide for tunable geometry and taperable angles, providing flexibility for accommodating varied anatomies and improves how grafts are fitted to a patient.

As used herein, "about" indicates a variance of up to 5% from the value being so modified. All values modified with "about" are also intended to convey the unmodified value as an alternative, so that "about 10 µm," by way of examples, discloses both a range of 9.5-10.5 µm as well as specifically 10 µm.

As used herein, "a stitched seam" indicates a sewn or sutured seam joining two woven textiles but does not encompass a transitional woven textile which joins two woven textiles by interlacing with the warp and weft thereof.

As used herein, "remote" indicates not directly adjacent to, either axially or circumferentially.

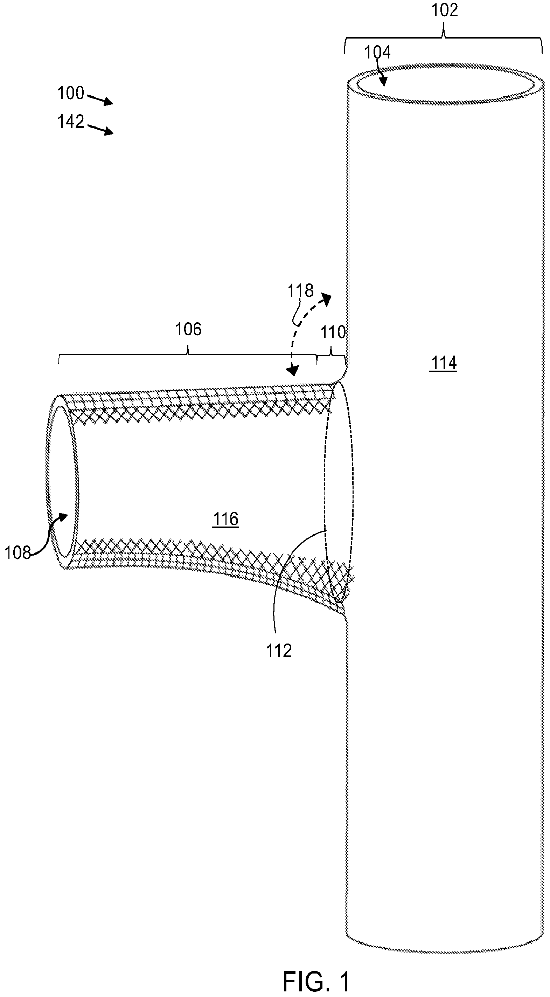

Referring to FIG. 1, in one embodiment, an integrally woven manifold 100 includes a primary portion 102 having a primary lumen 104 disposed therein, a secondary portion 106 branching off the primary portion 102, the secondary portion 106 having a secondary lumen 108 disposed therein in fluid communication with the primary lumen 104, and a first transitional portion 110 connecting the primary portion 102 to the secondary portion 106, the first transitional portion 110 having a first aperture 112 through which the secondary lumen 108 fluidly communicates with the primary lumen 104. The primary portion 102 has a first weave pattern 114, the secondary portion 106 has at least a second weave pattern 116, and the first transitional portion 110 is free of punctures, cuts, and bursts.

The first weave pattern 114 may be the same as the second weave pattern 116 or distinct from the second weave pattern 116. Suitable tubular weave patterns include, but are not limited to, tabby weave patterns, 2x2 twill weave patterns, 3x3 twill weave patterns, 4x1 twill weave patterns, 2x2 basket weave patterns, 2x1 weft/long rib weave patterns, 1x2 warp/cross rib weave patterns, crowsfoot weave patterns, 4x8 ripstop diamond weave patterns, or combinations thereof.

According the invention, a transition from the primary portion 102 to the secondary portion 106 through the first transitional portion 110 is free of stitched seams.

The secondary portion 106 extends from the primary portion 102 at a branching angle 118 between 75° to 105°, alternatively between 75° to 85°, alternatively between 80° to 90°, alternatively between 85° to 95°, alternatively between 90° to 100°, alternatively between 95° to 105°, any subrange thereof, or combinations thereof. In a further embodiment, the branching angle 118 is about 90°. The branching angle 118 is the angle at which the secondary portion 106 deviates from the primary portion 102 without kinking the primary portion 102, the secondary portion 106, or the first transitional portion 110.

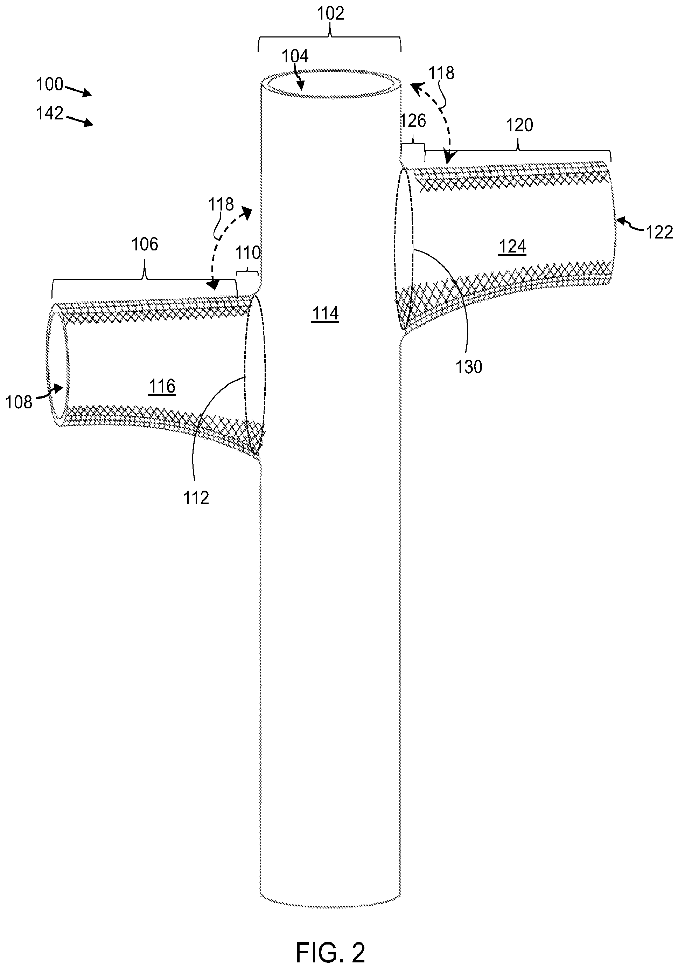

Referring to FIG. 2, in one embodiment, the integrally woven manifold 100 further includes a tertiary portion 120 branching off the primary portion 102, the tertiary portion 120 having a tertiary lumen 122 disposed therein in fluid communication with the primary lumen 104 and having a third weave pattern 124, the tertiary portion 120 being remote from the secondary portion 106, and a second transitional portion 126 connecting the primary portion 102 to the tertiary portion 120, the second transitional portion 126 having a second aperture 130 through which the tertiary lumen 122 fluidly communicates with the primary lumen 104. The second transitional portion 126 is free of punctures, cuts, and bursts. The third weave pattern 124 may be the same as the first weave pattern 114 or distinct from the first weave pattern 114. The third weave pattern 124 may be the same as the second weave pattern 116 or distinct from the second weave pattern 116.

In one embodiment, a transition from the primary portion 102 to the tertiary portion 120 through the second transitional portion 126 is free of stitched seams.

The tertiary portion 120 may extend from the primary portion 102 at any suitable branching angle 118, including, but not limited to, a branching angle 118 between 75° to 105°, alternatively between 75° to 85°, alternatively between 80° to 90°, alternatively between 85° to 95°, alternatively between 90° to 100°, alternatively between 95° to 105°, subranges thereof, or combinations thereof. In a further embodiment, the branching angle 118 is about 90°. The branching angle 118 is the angle at which the tertiary portion 120 deviates from the primary portion 102 without kinking the primary portion 102, the tertiary portion 120, or the second transitional portion 126.

The tertiary portion 120 may be radially aligned with the secondary portion 106 or may be offset about a periphery 128 of the primary portion 102 relative to the secondary portion 106.The offset about the periphery 128 may be any suitable offset, including, but not limited to, an offset between 5° to 180°, alternatively between 5° to 15°, alternatively between 10° to 20°, alternatively between 15° to 25°, alternatively between 20° to 30°, alternatively between 25° to 35°, alternatively between 30° to 40°, alternatively between 35° to 45°, alternatively between 40° to 50°, alternatively between 45° to 55°, alternatively between 50° to 60°, alternatively between 55° to 65°, alternatively between 60° to 70°, alternatively between 65° to 75°, alternatively between 70° to 80°, alternatively between 75° to 85°, alternatively between 80° to 90°, alternatively between 85° to 95°, alternatively between 90° to 100°, alternatively between 95° to 105°, alternatively between 100° to 110°, alternatively between 115° to 125°, alternatively between 120° to 130°, alternatively between 125° to 135°, alternatively between 130° to 140°, alternatively between 135° to 145°, alternatively between 140° to 150°, alternatively between 145° to 155°, alternatively between 150° to 160°, alternatively between 155° to 165°, alternatively between 160° to 170°, alternatively between 165° to 175°, alternatively between 170° to 180°, subranges thereof, or combinations thereof.

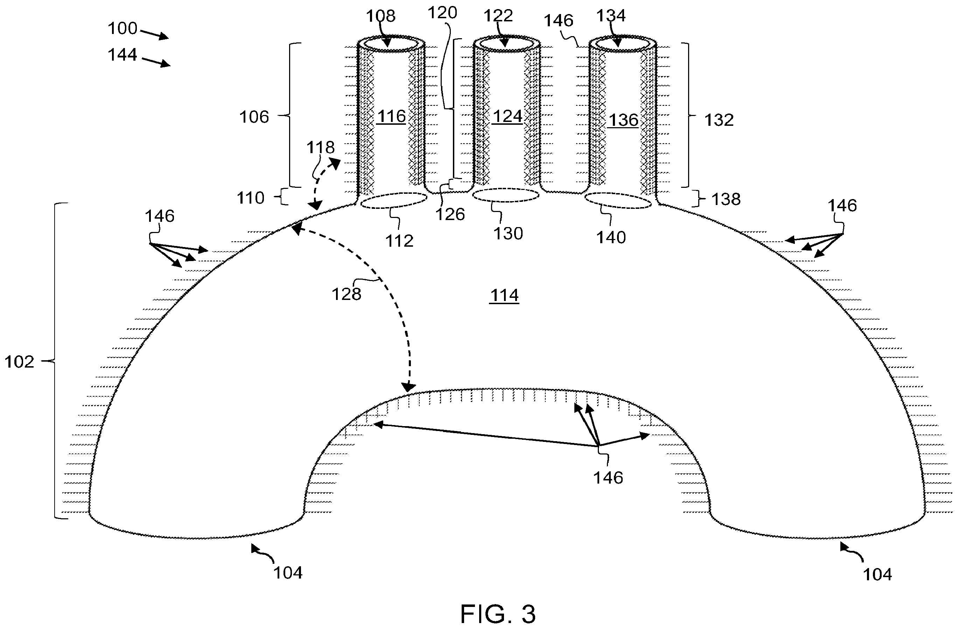

Referring to FIG. 3, in one embodiment, the integrally woven manifold 100 further includes a quaternary portion 132 branching off the primary portion 102, the quaternary portion 132 having a quaternary lumen 134 disposed therein in fluid communication with the primary lumen 104 and having a fourth weave pattern 136, the quaternary portion 132 being remote from the secondary portion 106 and tertiary portion 120, and a third transitional portion 138 connecting the primary portion 102 to the quaternary portion 132, the third transitional portion 138 having a third aperture 140 through which the quaternary lumen 134 fluidly communicates with the primary lumen 104. The third transitional portion 138 is free of punctures, cuts, and bursts.

The fourth weave pattern 136 may be the same as the first weave pattern 114 or distinct from the first weave pattern 114. The fourth weave pattern 136 may be the same as the second weave pattern 116 or distinct from the second weave pattern 116. The fourth weave pattern 136 may be the same as the third weave pattern 124 or distinct from the third weave pattern 124.

In one embodiment, a transition from the primary portion 102 to the quaternary portion 132 through the third transitional portion 138 is free of stitched seams.

The quaternary portion 132 may extend from the primary portion 102 at any suitable branching angle 118, including, but not limited to, a branching angle 118 between 75° to 105°, alternatively between 75° to 85°, alternatively between 80° to 90°, alternatively between 85° to 95°, alternatively between 90° to 100°, alternatively between 95° to 105°, subranges thereof, or combinations thereof. In a further embodiment, the branching angle 118 is about 90°. The branching angle 118 is the angle at which the quaternary portion 132 deviates from the primary portion 102 without kinking the primary portion 102, the quaternary portion 132, or the third transitional portion 138.

The quaternary portion 132 may be radially aligned with the secondary portion 106 or may be offset about a periphery 128 of the primary portion 102 relative to the secondary portion 106.The offset about the periphery 128 may be any suitable offset, including, but not limited to, an offset between 5° to 180°, alternatively between 5° to 15°, alternatively between 10° to 20°, alternatively between 15° to 25°, alternatively between 20° to 30°, alternatively between 25° to 35°, alternatively between 30° to 40°, alternatively between 35° to 45°, alternatively between 40° to 50°, alternatively between 45° to 55°, alternatively between 50° to 60°, alternatively between 55° to 65°, alternatively between 60° to 70°, alternatively between 65° to 75°, alternatively between 70° to 80°, alternatively between 75° to 85°, alternatively between 80° to 90°, alternatively between 85° to 95°, alternatively between 90° to 100°, alternatively between 95° to 105°, alternatively between 100° to 110°, alternatively between 115° to 125°, alternatively between 120° to 130°, alternatively between 125° to 135°, alternatively between 130° to 140°, alternatively between 135° to 145°, alternatively between 140° to 150°, alternatively between 145° to 155°, alternatively between 150° to 160°, alternatively between 155° to 165°, alternatively between 160° to 170°, alternatively between 165° to 175°, alternatively between 170° to 180°, subranges thereof, or combinations thereof.

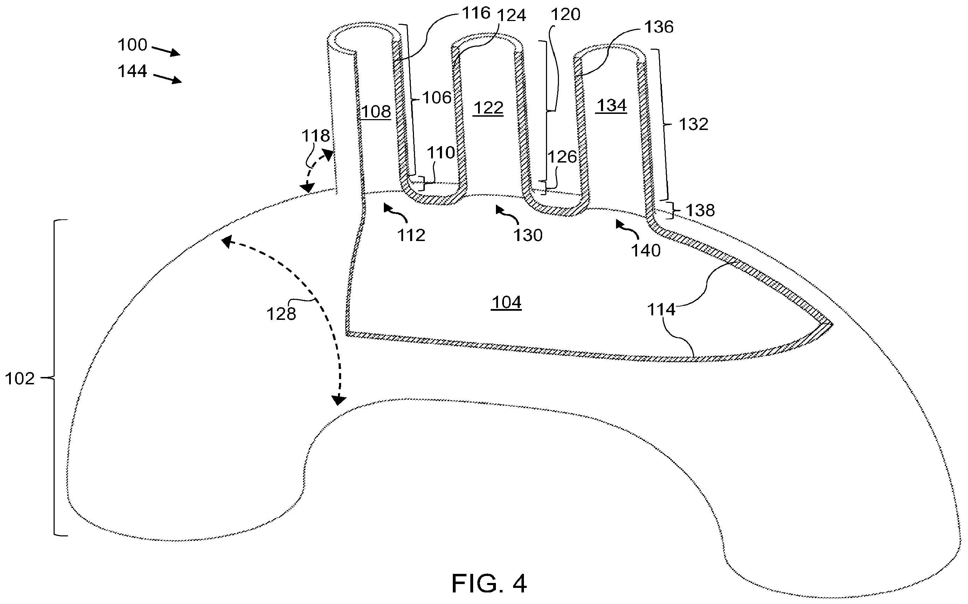

The integrally woven manifold 100 is a graft. Suitable grafts include an endovascular aneurysm repair graft 142 (FIGS. 1 and 2), an aortic root graft, an aortic arch graft 144 (FIGS. 3 and 4), an endoprosthesis stent graft, or a Circle of Willis graft.

Referring to FIGS. 1 and 2, in one embodiment, integrally woven manifold 100 is an all-in-one endovascular aneurysm repair graft that has both a primary lumen 104 for the aorta and smaller lumen extensions 108/122 that reach out from the primary lumen 104 as one continuous textile graft 100. In one embodiment wherein the integrally woven manifold 100 is a branched graft, the branched graft may be configured to mimic any suitable organ. Weaving a customized branched graft with multiple branches positioned independently at predetermined angles from the primary lumen 104 supports biomimetic design.

An integrally woven manifold 100 may reproduce a full endoprosthesis, such as from valve through the aortic arch 144, which may be suitable for integrated coronary access. Weaving a customized integrally woven manifold 100 as a branched graft with multiple branches positioned independently at predetermined angles from the primary lumen 104, supporting biomimetic design, may form a fully integrated, endovascular replacement valve/graft system 142. The branched graft may endovascularly provide access to the coronary arteries, offering a solution for coronary obstruction from transcatheter aortic valve replacement and native leaflet interference. An endoprosthesis stent graft coming off the top of a transcatheter aortic valve replacement device with branched lumens for coronary access may eliminate the potential for the native leaflets to block these vessels by routing the blood flow up and around the leaflets. Integrally woven branched grafts formed as biomimetic constructs may also be suitable for lung surgery and peripheral vascular repair, or for forming circular lumens and branches such as for forming biomimetic constructs for anatomy such as the Circle of Willis at the base of the skill which may be damaged from head trauma.

Any of the first transitional portion 110, the second transitional portion 126, or the third transitional portion 138 may taper from the primary portion 102, to the secondary portion 106, to the tertiary portion 120, or to the quaternary portion 132, respectively, or may have a constant diameter from the primary portion 102 to the secondary portion 106, to the tertiary portion 120, or to the quaternary portion 132, respectively, or any combination thereof.

Any of the first transitional portion 110, the second transitional portion 126, or the third transitional portion 138, or any combination thereof, may include an elastomeric material which increases pulsatile behavior relative to a comparative transitional portion which is otherwise identical but lacking the elastomeric material. In a further embodiment, the primary portion 102, the secondary portion 106, the tertiary portion 120 if present, and the quaternary portion 132 if present are free of the elastomeric material.

In one embodiment, the primary portion 102 and the secondary portion 106 are composed with different warp yarns. In a further embodiment, the tertiary portion 120 is composed with different warp yarns than the primary portion 102 and either the same or different warp yarns as the secondary portion 106. In yet a further embodiment, the quaternary portion 132 is composed with different warp yarns than the primary portion 102 and either the same or different warp yarns as either or both of the secondary portion 106 or the tertiary portion 120.

The primary portion 102 and the secondary portion 106 may have the same number of warp yarns or a different number of warp yarns. If present, the tertiary portion 120 may have the same number or warp yarns or a different number of warp yarns as either or both of the primary portion 102 or the secondary portion 106. If present, the quaternary portion 132 may have the same number or warp yarns or a different number of warp yarns as any of, any two of, or all of the primary portion 102, the secondary portion 106, or the tertiary portion 120.

In one embodiment, the integrally woven manifold 100 is free of diameter control weaves in which a second weft yarn is interlaced with segments of at least a portion of the warp yarns outside of the integrally woven manifold 100.

In one embodiment, the primary portion 102 is free of a ring of dropped yarns spaced apart around the circumference of the primary portion 102. In a further embodiment, the seconary portion 106 is free of a ring of dropped yarns spaced apart around the circumference of the secondary portion 106. In a still further embodiment, the tertiary portion 120, if present, is free of a ring of dropped yarns spaced apart around the circumference of the tertiary portion 120 and the quaternary portion 132, if present, is free of a ring of dropped yarns spaced apparat around the circumference of the quaternary portion 132. In yet a further embodiment, the first transitional portion 110 is free of a ring of dropped yarns spaced apart around the circumference of the first transitional portion 110, the second transitional portion 126, if present, is free of a ring of dropped yarns spaced apart around the circumference of the second transitional portion 126, and the third transitional portion 138, if present, is free of a ring of dropped yarns spaced apart around the circumference of the third transitional portion 138.

In one embodiment, the secondary portion 106, and, if present, the tertiary portion 120 and the quaternary portion 132 branch off of the primary portion 102 via fenestration rather than branching off via bifurcation of the primary portion 102. In a further embodiment, the integrally woven manifold 100 is free of bifurcated branching in which a single tube is divided into two or more smaller tubes.

Any of the primary portion 102, the secondary portion 106, the tertiary portion 120, the quaternary portion 132, the first transitional portion 110, the second transitional portion 126, or the third transitional portion 138, or combinations thereof, may be formed at least partially from resorbable materials. The resorbable materials may be present in the form of the warp, the weft, coatings on the warp, coatings on the weft, coatings on the integrally woven manifold 100, or combinations thereof. The resorbable materials may be any suitable materials, including, but not limited to, polyglycolide, poly(lactic-co-glycolic acid), polylactic acid, polycaprolactone, poly(glycerol sebacate), poly(glycerol sebacate) urethane, or combinations thereof. Additional biologic and synthetic materials, or combinations thereof, may further be used in combination with the resorbable materials so as to form endogenously regenerative grafts.

In one embodiment, the first weave pattern 114, the second weave pattern 116, the third weave pattern 124, and the fourth weave pattern 136 are independently selected from suitable weave patterns to provide different textile characteristics for different predetermined portions of the integrally woven manifold 100, such as, but not limited to, selecting a weave pattern providing increased radial strength in the first transitional portion 110, the second transitional portion 126, the third transitional portion 138, or combinations thereof relative to the primary portion 102, the secondary portion 106, to the tertiary portion 120, or to the quaternary portion 132.

The integrally woven manifold 100 may include any number of additional portions with lumens beyond the secondary portion 106, the tertiary portion 120, and the quaternary portion 132 with the same features, including a fifth, a sixth, a seventh, an eight, a ninth, a tenth or more portions.

Referring to FIG. 1, in one embodiment, a tubular tabby weave may be used for the secondary portion 106, the tertiary portion 120, the quaternary portion 132, or combinations thereof in combination with a ripstop weave (tubular or flat) in a tapering portion of the secondary portion 106, the tertiary portion 120, the quaternary portion 132, or combinations thereof, and with a tabby weave or weft rib weave to seal close an arm extension on the top and bottom of the secondary portion 106, the tertiary portion 120, the quaternary portion 132, or combinations thereof. The tubular tabby weave may provide a fluid impermeable lumen center while the transitioning weave may bridge the interlacement jump from a tubular weave to a flat weave. A standard tubular weave will typically have 50% of its ends forming a first side of the tube and the other 50% of the ends forming the opposing second side of the tube. As such, bringing together both sets of the tubular weave into a flat weave would increase weave density by 100%. A transition weave may provide a middle density which eases tensions of the warp ends and promotes weaving efficiency.

Referring to FIGS. 3 and 4, in one embodiment, a method for forming an integrally woven manifold 100 includes continuously interlacing warp and weft to form the primary portion 102, intermittently interlacing the warp and the weft to form the secondary portion 106 (and optionally the tertiary portion 120 and optionally the quaternary portion 132) branching off the primary portion 102, and disengaging and reengaging the warp and the weft to form the first transitional portion 110 (and optionally the second transitional portion 126 and optionally the third transitional portion 138). Disengaging and reengaging the warp and the weft to form the first transitional portion 110 (and optionally the second transitional portion 126 and optionally the third transitional portion 138) may form the first transitional portion 110 (and optionally the second transitional portion 126 and optionally the third transitional portion 138) free of stitched seams.

The warp ends disengaged from the weft during the formation of the first transitional portion 110 (and optionally the second transitional portion 126 and optionally the third transitional portion 138) may be left floating or may be used to weave a separate independent textile.

When the warp and weft are engaged to form the first transitional portion 110 (and optionally the second transitional portion 126 and optionally the third transitional portion 138) as well as the secondary portion 106 (and optionally the tertiary portion 120 and optionally the quaternary portion 132), the warp may be interlaced with the weft in suitable patterns to maintain a consistent density. The first transitional portion 110 (and optionally the second transitional portion 126 and optionally the third transitional portion 138) may be interlaced such that a fluid such as blood may flow freely from the primary portion 102 through the first transitional portion 110 (and optionally the second transitional portion 126 and optionally the third transitional portion 138) and then through the secondary portion 106 (and optionally the tertiary portion 120 and optionally the quaternary portion 132) (or the flow direction may be reversed).

After the integrally woven manifold 100 is woven, the integrally woven manifold 100 may be subjected to any suitable post-processing, including, but not limited to, scouring, drying, heat pressing, or combinations thereof. Such post-processing may enhance the dimensional stability of the integrally woven manifold 100.

The floating ends 146, and separate independent textile, if present, may be removed from the integrally woven manifold 100.

This method may be used to form any suitable number of arms and arm branches for a graft in order to mimic vascular (arterial and venous) anatomy, including, but not limited to, two arm extensions, three arm extensions, four arm extensions, five arm extensions, or more. Further, the angle of the arm extensions relative to the primary lumen 104 may be modified by adjusting the weave pattern so as to mimic native anatomy.

The method may be performed any suitable number of shuttles, including a single shuttle, two shuttles, three shuttles, four shuttles, five shuttles, or more.

While the foregoing specification illustrates and describes exemplary embodiments, it will be understood by those skilled in the art that various changes may be made and equivalents may be substituted for elements thereof without departing from the scope of the appended claims. In addition, many modifications may be made to adapt a particular situation or material to the teachings of the invention without departing from the essential scope of the appended claims. Therefore, it is intended that the invention not be limited to the particular embodiment disclosed as the best mode contemplated for carrying out this invention, but that the invention will include all embodiments falling within the scope of the appended claims.

## Claims

1. An integrally woven manifold (100), comprising:
a primary portion (102) having a primary lumen (104) disposed therein;
a secondary portion (106) extending from and branching off the primary portion (102), the secondary portion (106) having a secondary lumen (108) disposed therein in fluid communication with the primary lumen (104); and
a first transitional portion (110) connecting the primary portion (102) to the secondary portion (106), the first transitional portion (110) having a first aperture (112) through which the secondary lumen (108) fluidly communicates with the primary lumen (104),
wherein:
the integrally woven manifold (100) is a graft selected from the group consisting of an endovascular aneurysm repair graft (142), an aortic roof graft, an aortic arch graft (144), an endoprosthesis stent graft, and a Circle of Willis graft;
each of the primary portion (102) and the secondary portion (106) mimics vascular anatomy;
the primary portion (102) has a first weave pattern (114);
the secondary portion (106) has at least a second weave pattern (116);
the first transitional portion (110) is free of punctures, cuts, and bursts; and
a transition from the primary portion (102) to the secondary portion (106) through the first transitional portion (110) is free of stitched seams, **characterized in that** the secondary portion (106) branches off the primary portion (102) at a branching angle between 75° to 105°.

2. The integrally woven manifold (100) of claim 1, wherein the first weave pattern (114) is distinct from the second weave pattern (116).

3. The integrally woven manifold (100) of claim 1, further including:
a tertiary portion (120) branching off the primary portion (102), the tertiary portion (120) having a tertiary lumen (122) disposed therein in fluid communication with the primary lumen (104) and having a third weave pattern (124), the tertiary portion (120) being remote from the secondary portion (106); and
a second transitional portion (126) connecting the primary portion (102) to the tertiary portion (120), the second transitional portion (126) having a second aperture (130) through which the tertiary lumen (122) fluidly communicates with the primary lumen (104),
wherein the second transitional portion (126) is free of punctures, cuts, and bursts.

4. The integrally woven manifold (100) of claim 3, wherein the third weave pattern (124) is distinct from the first weave pattern (114), preferably wherein the third weave pattern (124) is distinct from the second weave pattern (116), or more preferably wherein a transition from the primary portion (102) to the tertiary portion (120) through the second transitional portion (126) is free of stitched seams.

5. The integrally woven manifold (100) of claim 3, wherein the tertiary portion (120) extends from the primary portion (102) at a branching angle between 75° to 105°, preferably wherein the tertiary portion (120) is radially offset about a periphery of the primary portion (102) relative to the secondary portion (106).

6. The integrally woven manifold (100) of claim 3, further including:
a quaternary portion (132) branching off the primary portion (102), the quaternary portion (132) having a quaternary lumen (134) disposed therein in fluid communication with the primary lumen (104) and having a fourth weave pattern (136), the quaternary portion (132) being remote from the secondary portion (106) and tertiary portion (120); and
a third transitional portion (138) connecting the primary portion (102) to the quaternary portion (132), the third transitional portion (138) having a third aperture through which the quaternary lumen (134) fluidly communicates with the primary lumen (102),
wherein the third transitional portion (138) is free of punctures, cuts, and bursts.

7. The integrally woven manifold (100) of claim 1, wherein the integrally woven manifold (100) is the aortic root graft or the aortic arch graft (144).

8. The integrally woven manifold (100) of claim 1, wherein the integrally woven manifold (100) is the Circle of Willis graft.

9. The integrally woven manifold (100) of claim 1, wherein the first transitional portion (110) tapers from the primary portion (102) to the secondary portion (106).

10. The integrally woven manifold (100) of claim 1, wherein the first transitional portion (110) includes an elastomeric material which increases pulsatile behavior relative to a comparative transitional portion which is otherwise identical but lacking the elastomeric material, preferably wherein the primary portion (102) and the secondary portion (106) are free of the elastomeric material.

11. The integrally woven manifold (100) of claim 1, wherein the primary portion (102), the secondary portion (106), and the first transitional portion (110) are formed at least partially from resorbable materials selected from the group consisting of polyglycolide, poly(lactic-co-glycolic acid), polylactic acid, polycaprolactone, poly(glycerol sebacate), poly(glycerol sebacate) urethane, or combinations thereof.

12. The integrally woven manifold (100) of claim 1, wherein the primary portion (102) and the secondary portion (106) are composed with different warp yarns.

13. A method for forming an integrally woven manifold (100), comprising:
continuously interlacing warp and weft to form a primary portion (102) having a primary lumen (104) disposed therein;
intermittently interlacing the warp and the weft to form a secondary portion (106) extending from and branching off the primary portion (102), the secondary portion (106) having a secondary lumen (108) disposed therein in fluid communication with the primary lumen (104); and
disengaging and reengaging the warp and the weft to form a first transitional portion (110) connecting the primary portion (102) to the secondary portion (106), the first transitional portion (110) having a first aperture (112) through which the secondary lumen (108) fluidly communicates with the primary lumen (104),
wherein:
the integrally woven (100) manifold is a graft selected from the group consisting of an endovascular aneurysm repair graft (142), an aortic roof graft, an aortic arch graft (144), an endoprosthesis stent graft, and a Circle of Willis graft;
each of the primary portion (102) and the secondary portion (106) mimics vascular anatomy ;
the primary portion (102) has a first weave pattern (114);
the secondary portion (106) has at least a second weave pattern (116);
the first transitional portion (110) is free of punctures, cuts, and bursts; and
a transition from the primary portion (102) to the secondary portion (106) through the first transitional portion (110) is free of stitched seams, **characterized in that** the secondary portion (106) branches off the primary portion (102) at a branching angle between 75° to 105°.

14. The method of claim 13, wherein disengaging and reengaging the warp and the weft to form the first transitional portion (110) forms the first transitional portion (110) free of stitched seams.

## Patentansprüche

1. Einstückig gewebter Verteiler (100), umfassend:
einen primären Abschnitt (102) mit einem darin angeordneten primären Lumen (104),
einen sekundären Abschnitt (106), der sich ausgehend vom primären Abschnitt (102) erstreckt und von diesem abzweigt, wobei der sekundäre Abschnitt (106) ein darin angeordnetes sekundäres Lumen (108) aufweist, das in Strömungsverbindung mit dem primären Lumen (104) steht, und
einen ersten Übergangsabschnitt (110), der den primären Abschnitt (102) mit dem sekundären Abschnitt (106) verbindet, wobei der erste Übergangsabschnitt (110) eine erste Öffnung (112) aufweist, durch die das sekundäre Lumen (108) in Strömungsverbindung mit dem primären Lumen (104) steht,
wobei:
der einstückig gewebte Verteiler (100) ein Transplantat ist, das aus der Gruppe ausgewählt ist, die aus einem endovaskulären Aneurysma-Reparaturtransplantat (142), einem Aortenwurzeltransplantat, einem Aortenbogentransplantat (144), einem Endoprothesen-Stentgraft und einem Transplantat des Circulus (arteriosus) Willisi besteht,
sowohl der primäre Abschnitt (102) als auch der sekundäre Abschnitt (106) die Gefäßanatomie nachbilden,
der primäre Abschnitt (102) ein erstes Webmuster (114) aufweist,
der sekundäre Abschnitt (106) mindestens ein zweites Webmuster (116) aufweist,
der erste Übergangsabschnitt (110) frei von Einstichen, Schnitten und Rissen ist und
ein Übergang vom primären Abschnitt (102) zum sekundären Abschnitt (106) über den ersten Übergangsabschnitt (110) frei von Nahtverbindungen ist,
**dadurch gekennzeichnet, dass** der sekundäre Abschnitt (106) unter einem Abzweigwinkel zwischen 75° und 105° vom primären Abschnitt (102) abzweigt.

2. Einstückig gewebter Verteiler (100) nach Anspruch 1, wobei sich das erste Webmuster (114) vom zweiten Webmuster (116) unterscheidet.

3. Einstückig gewebter Verteiler (100) nach Anspruch 1, ferner umfassend:
einen vom primären Abschnitt (102) abzweigenden tertiären Abschnitt (120), wobei der tertiäre Abschnitt (120) ein darin angeordnetes tertiäres Lumen (122) aufweist, das in Strömungsverbindung mit dem primären Lumen (104) steht, und ein drittes Webmuster (124) aufweist, wobei der tertiäre Abschnitt (120) vom sekundären Abschnitt (106) entfernt ist, und
einen zweiten Übergangsabschnitt (126), der den primären Abschnitt (102) mit dem tertiären Abschnitt (120) verbindet, wobei der zweite Übergangsabschnitt (126) eine zweite Öffnung (130) aufweist, durch die das tertiäre Lumen (122) in Strömungsverbindung mit dem primären Lumen (104) steht,
wobei der zweite Übergangsabschnitt (126) frei von Einstichen, Schnitten und Rissen ist.

4. Einstückig gewebter Verteiler (100) nach Anspruch 3, wobei sich das dritte Webmuster (124) vom ersten Webmuster (114) unterscheidet, vorzugsweise wobei sich das dritte Webmuster (124) vom zweiten Webmuster (116) unterscheidet, oder weiter vorzugsweise wobei ein Übergang vom primären Abschnitt (102) zum tertiären Abschnitt (120) über den zweiten Übergangsabschnitt (126) frei von Nahtverbindungen ist.

5. Einstückig gewebter Verteiler (100) nach Anspruch 3, wobei sich der tertiäre Abschnitt (120) unter einem Abzweigwinkel zwischen 75° und 105° ausgehend vom primären Abschnitt (102) erstreckt, vorzugsweise wobei der tertiäre Abschnitt (120) um einen Umfang des primären Abschnitts (102) bezogen auf den sekundären Abschnitt (106) radial versetzt ist.

6. Einstückig gewebter Verteiler (100) nach Anspruch 3, ferner umfassend:
einen vom primären Abschnitt (102) abzweigenden quartären Abschnitt (132), wobei der quartäre Abschnitt (132) ein darin angeordnetes quartäres Lumen (134) aufweist, das in Strömungsverbindung mit dem primären Lumen (104) steht, und ein viertes Webmuster (136) aufweist, wobei der quartäre Abschnitt (132) vom sekundären Abschnitt (106) und vom tertiären Abschnitt (120) entfernt ist, und
einen dritten Übergangsabschnitt (138), der den primären Abschnitt (102) mit dem quartären Abschnitt (132) verbindet, wobei der dritte Übergangsabschnitt (138) eine dritte Öffnung aufweist, durch die das quartäre Lumen (134) in Strömungsverbindung mit dem primären Lumen (102) steht,
wobei der dritte Übergangsabschnitt (138) frei von Einstichen, Schnitten und Rissen ist.

7. Einstückig gewebter Verteiler (100) nach Anspruch 1, wobei es sich bei dem einstückig gewebten Verteiler (100) um das Aortenwurzeltransplantat oder das Aortenbogentransplantat (144) handelt.

8. Einstückig gewebter Verteiler (100) nach Anspruch 1, wobei es sich bei dem einstückig gewebten Verteiler (100) um das Transplantat des Circulus (arteriosus) Willisi handelt.

9. Einstückig gewebter Verteiler (100) nach Anspruch 1, wobei sich der erste Übergangsabschnitt (110) vom primären Abschnitt (102) zum sekundären Abschnitt (106) verjüngt.

10. Einstückig gewebter Verteiler (100) nach Anspruch 1, wobei der erste Übergangsabschnitt (110) ein Elastomermaterial enthält, das im Vergleich zu einem ansonsten identischen, jedoch ohne das Elastomermaterial ausgebildeten Vergleichs-Übergangsabschnitt das pulsatile Verhalten erhöht, wobei vorzugsweise der primäre Abschnitt (102) und der sekundäre Abschnitt (106) frei von dem Elastomermaterial sind.

11. Einstückig gewebter Verteiler (100) nach Anspruch 1, wobei der primäre Abschnitt (102), der sekundäre Abschnitt (106) und der erste Übergangsabschnitt (110) zumindest teilweise aus resorbierbaren Materialien gebildet sind, die aus der Gruppe ausgewählt sind, die aus Polyglykolid, Poly(lactid-co-glycolid), Polylactid, Polycaprolacton, Poly(glycerolsebacat), Poly(glycerolsebacat)-Urethan oder Kombinationen davon besteht.

12. Einstückig gewebter Verteiler (100) nach Anspruch 1, wobei der primäre Abschnitt (102) und der sekundäre Abschnitt (106) aus unterschiedlichen Kettfäden bestehen.

13. Verfahren zur Herstellung eines einstückig gewebten Verteilers (100) (100), das Folgendes umfasst:
kontinuierliches Verflechten von Kett- und Schussfäden zur Bildung eines primären Abschnitts (102) mit einem darin angeordneten primären Lumen (104),
intermittierendes Verflechten von Kett- und Schussfäden zur Bildung eines sekundären Abschnitts (106), der sich ausgehend vom primären Abschnitt (102) erstreckt und von diesem abzweigt, wobei der sekundäre Abschnitt (106) ein darin angeordnetes sekundäres Lumen (108) aufweist, das in Strömungsverbindung mit dem primären Lumen (104) steht, und
Trennen und erneutes Verschlingen der Kett- und Schussfäden zur Bildung eines ersten Übergangsabschnitts (110), der den primären Abschnitt (102) mit dem sekundären Abschnitt (106) verbindet, wobei der erste Übergangsabschnitt (110) eine erste Öffnung (112) aufweist, durch die das sekundäre Lumen (108) in Strömungsverbindung mit dem primären Lumen (104) steht,
wobei:
der einstückig gewebte Verteiler (100) ein Transplantat ist, das aus der Gruppe ausgewählt ist, die aus einem endovaskulären Aneurysma-Reparaturtransplantat (142), einem Aortenwurzeltransplantat, einem Aortenbogentransplantat (144), einem Endoprothesen-Stentgraft und einem Transplantat des Circulus (arteriosus) Willisi besteht,
sowohl der primäre Abschnitt (102) als auch der sekundäre Abschnitt (106) die Gefäßanatomie nachbilden,
der primäre Abschnitt (102) ein erstes Webmuster (114) aufweist,
der sekundäre Abschnitt (106) mindestens ein zweites Webmuster (116) aufweist,
der erste Übergangsabschnitt (110) frei von Einstichen, Schnitten und Rissen ist und
ein Übergang vom primären Abschnitt (102) zum sekundären Abschnitt (106) über den ersten Übergangsabschnitt (110) frei von Nahtverbindungen ist,
**dadurch gekennzeichnet, dass** der sekundäre Abschnitt (106) unter einem Abzweigwinkel zwischen 75° und 105° vom primären Abschnitt (102) abzweigt.

14. Verfahren nach Anspruch 13, wobei durch Trennen und erneutes Verschlingen der Kett- und Schussfäden zur Bildung des ersten Übergangsabschnitts (110) der erste Übergangsabschnitt (110) frei von Nahtverbindungen gebildet wird.

## Revendications

1. Collecteur (100) tissé d'un seul tenant, comprenant :
une partie primaire (102) présentant une lumière primaire (104) disposée dans celle-ci;
une partie secondaire (106) s'étendant et bifurquant à partir de la partie primaire (102), la partie secondaire (106) présentant une lumière secondaire (108) disposée dans celle-ci et en communication fluidique avec la lumière primaire (104) ; et
une première partie de transition (110) reliant la partie primaire (102) à la partie secondaire (106), la première partie de transition (110) présentant une première ouverture (112) à travers laquelle la lumière secondaire (108) communique fluidiquement avec la lumière primaire (104),
dans lequel :
le collecteur (100) tissé d'un seul tenant est un greffon choisi dans le groupe constitué d'un greffon endovasculaire de réparation d'anévrisme (142), d'un greffon de racine d'aorte, d'un greffon d'arc aortique (144), d'un greffon-stent endoprothétique et d'un greffon du cercle de Willis ;
la partie primaire (102) et la partie secondaire (106) imitent chacune l'anatomie vasculaire ;
la partie primaire (102) présente un premier motif de tissage (114) ;
la partie secondaire (106) présente au moins un deuxième motif de tissage (116) ;
la première partie de transition (110) est exempte de perforations, de coupures et de ruptures ; et
une transition de la partie primaire (102) à la partie secondaire (106) via la première partie de transition (110) est exempte de coutures,
**caractérisé en ce que** la partie secondaire (106) bifurque à partir de la partie primaire (102) selon un angle de bifurcation compris entre 75° et 105°.

2. Collecteur (100) tissé d'un seul tenant selon la revendication 1, dans lequel le premier motif de tissage (114) est distinct du deuxième motif de tissage (116).

3. Collecteur (100) tissé d'un seul tenant selon la revendication 1, comprenant en outre :
une partie tertiaire (120) bifurquant à partir de la partie primaire (102), la partie tertiaire (120) présentant une lumière tertiaire (122) disposée dans celle-ci et en communication fluidique avec la lumière primaire (104), et présentant un troisième motif de tissage (124), la partie tertiaire (120) étant éloignée de la partie secondaire (106) ; et
une deuxième partie de transition (126) reliant la partie primaire (102) à la partie tertiaire (120), la deuxième partie de transition (126) présentant une deuxième ouverture (130) à travers laquelle la lumière tertiaire (122) communique fluidiquement avec la lumière primaire (104),
la deuxième partie de transition (126) étant exempte de perforations, de coupures et de ruptures.

4. Collecteur (100) tissé d'un seul tenant selon la revendication 3, dans lequel le troisième motif de tissage (124) est distinct du premier motif de tissage (114), de préférence dans lequel le troisième motif de tissage (124) est distinct du deuxième motif de tissage (116), ou plus préférablement dans lequel une transition de la partie primaire (102) à la partie tertiaire (120) via la deuxième partie de transition (126) est exempte de coutures.

5. Collecteur (100) tissé d'un seul tenant selon la revendication 3, dans lequel la partie tertiaire (120) s'étend à partir de la partie primaire (102) selon un angle de bifurcation compris entre 75° et 105°, de préférence dans lequel la partie tertiaire (120) est décalée radialement autour d'une périphérie de la partie primaire (102) par rapport à la partie secondaire (106).

6. Collecteur (100) tissé d'un seul tenant selon la revendication 3, comprenant en outre :
une partie quaternaire (132) bifurquant à partir de la partie primaire (102), la partie quaternaire (132) présentant une lumière quaternaire (134) disposée dans celle-ci et en communication fluidique avec la lumière primaire (104), et présentant un quatrième motif de tissage (136), la partie quaternaire (132) étant éloignée de la partie secondaire (106) et de la partie tertiaire (120) ; et
une troisième partie de transition (138) reliant la partie primaire (102) à la partie quaternaire (132), la troisième partie de transition (138) présentant une troisième ouverture à travers laquelle la lumière quaternaire (134) communique fluidiquement avec la lumière primaire (102),
la troisième partie de transition (138) étant exempte de perforations, de coupures et de ruptures.

7. Collecteur (100) tissé d'un seul tenant selon la revendication 1, dans lequel le collecteur (100) tissé d'un seul tenant est le greffon de racine aortique ou le greffon d'arc aortique (144).

8. Collecteur (100) tissé d'un seul tenant selon la revendication 1, dans lequel le collecteur (100) tissé d'un seul tenant est le greffon du cercle de Willis.

9. Collecteur (100) tissé d'un seul tenant selon la revendication 1, dans lequel la première partie de transition (110) se rétrécit de la partie primaire (102) vers la partie secondaire (106).

10. Collecteur (100) tissé d'un seul tenant selon la revendication 1, dans lequel la première partie de transition (110) comprend un matériau élastomère qui augmente le comportement pulsatile par rapport à une partie de transition comparative qui est par ailleurs identique mais dépourvue du matériau élastomère, de préférence dans lequel la partie primaire (102) et la partie secondaire (106) sont exemptes du matériau élastomère.

11. Collecteur (100) tissé d'un seul tenant selon la revendication 1, dans lequel la partie primaire (102), la partie secondaire (106) et la première partie de transition (110) sont réalisées au moins partiellement à partir de matériaux résorbables choisis dans le groupe constitué de polyglycolide, de poly(acide lactique-co-glycolique), d'acide polylactique, de polycaprolactone, de poly(sébacate de glycérol), de poly(sébacate de glycérol)uréthane, ou des combinaisons de ceux-ci.

12. Collecteur (100) tissé d'un seul tenant selon la revendication 1, dans lequel la partie primaire (102) et la partie secondaire (106) sont composées de fils de chaîne différents.

13. Procédé de réalisation d'un collecteur (100) tissé d'un seul tenant, comprenant :
l'entrelacement continu de la chaîne et de la trame pour former une partie primaire (102) présentant une lumière primaire (104) disposée dans celle-ci ;
l'entrelacement intermittent de la chaîne et de la trame pour former une partie secondaire (106) s'étendant et bifurquant à partir de la partie primaire (102), la partie secondaire (106) présentant une lumière secondaire (108) disposée dans celle-ci et en communication fluidique avec la lumière primaire (104) ; et
le désengagement puis le réengagement de la chaîne et de la trame pour former une première partie de transition (110) reliant la partie primaire (102) à la partie secondaire (106), la première partie de transition (110) présentant une première ouverture (112) à travers laquelle la lumière secondaire (108) communique fluidiquement avec la lumière primaire (104),
dans lequel :
le collecteur (100) tissé d'un seul tenant est un greffon choisi dans le groupe constitué d'un greffon endovasculaire de réparation d'anévrisme (142), d'un greffon de racine d'aorte, d'un greffon d'arc aortique (144), d'un greffon-stent endoprothétique et d'un greffon du cercle de Willis ;
la partie primaire (102) et la partie secondaire (106) imitent chacune l'anatomie vasculaire ;
la partie primaire (102) présente un premier motif de tissage (114) ;
la partie secondaire (106) présente au moins un deuxième motif de tissage (116) ;
la première partie de transition (110) est exempte de perforations, de coupures et de ruptures ; et
une transition de la partie primaire (102) à la partie secondaire (106) via la première partie de transition (110) est exempte de coutures,
**caractérisé en ce que** la partie secondaire (106) bifurque à partir de la partie primaire (102) selon un angle de bifurcation compris entre 75° et 105°.

14. Procédé selon la revendication 13, dans lequel le désengagement et le réengagement de la chaîne et de la trame pour former la première partie de transition (110) forment la première partie de transition (110) sans coutures.
